# EUROPEAN PATENT SPECIFICATION

(11) **EP 3 299 011 B1**
(45) Date of publication and mention of the grant of the patent: **18.08.2021**
(21) Application number: 17192733.8
(22) Date of filing: 22.09.2017
(51) Int. Cl.: A61K 9/08, A61K 47/42, A61K 47/02, A61K 31/191, A61K 31/194, A61K 31/575, A61K 33/14, A61K 38/47, A61P 3/04

(54) **HYPOTONIC COMPOSITION FOR FAT DECOMPOSITION AND METHOD FOR PREPARING SAME**
HYPOTONE ZUSAMMENSETZUNG ZUR FETTZERSETZUNG UND VERFAHREN ZUR HERSTELLUNG DAVON
COMPOSITION HYPOTONIQUE DÉCOMPOSITION DE GRAISSE ET SON PROCÉDÉ DE PRÉPARATION

(30) Priority: 22.09.2016 KR 20160121404; 12.04.2017 KR 20170047435
(43) Date of publication of application: 28.03.2018
(73) Proprietor: Lee, Kyoung Lack, Seoul 06217 (KR); Kang, Si Ha, Seoul 04722 (KR)
(72) Inventor: Lee, Kyoung Lack, Seoul 06217 (KR); Kang, Si Ha, Seoul 04722 (KR)
(74) Representative: McDade, Sophie V.G.A.

(56) References cited:
- CA-A1- 2 872 279
- US-A1- 2005 267 080
- PETER M CRAPO ET AL: "An overview of tissue and whole organ decellularization processes", BIOMATERIALS, ELSEVIER SCIENCE PUBLISHERS BV., BARKING, GB, vol. 32, no. 12, 19 January 2011 (2011-01-19), pages 3233-3243, XP028149166, ISSN: 0142-9612, DOI: 10.1016/J.BIOMATERIALS.2011.01.057 [retrieved on 2011-01-25]

## Description

### Technical Field

The present invention relates to a hypotonic composition for fat decomposition and a method for preparing the same and, specifically, to a hypotonic composition for fat decomposition and a method for preparing the same, wherein a hypotonic composition for fat decomposition is prepared to contain sodium deoxycholate, hyaluronidase, a buffer, an additive, and the like in a hypotonic solution composed of distilled water and sodium chloride.

### Background Art

In general, for the purpose of lipoplasty, a complex preparation injection using phosphatidylcholine (PPC) and sodium deoxycholate has been widely used for topical obesity treatment. See, for example, patent applications US 2005/267080A1 (KOLODNEY MICHAEL ET AL, ) and CA 2872279A1 (MODI PANKAJ)and scientific article CRAPO ET AL: "An overview of 1-9 tissue and whole organ decellularization processes", BIOMATERIALS, ELSEVIER SCIENCE PUBLISHERS, vol. 32, no. 12, 19 January 2011 (2011-01-19), pages 3233-3243.

Since fat necrosis occurs due to such a complex preparation injection using phosphatidylcholine (PPC) and sodium deoxycholate, a composition for fat decomposition using a preparation of sodium deoxycholate alone excluding phosphatidylcholine has been developed. However, the composition for fat decomposition using a preparation of sodium deoxycholate alone frequently causes adverse effects, such as edema, erythema, pain, and callosity.

### Detailed Description of the Invention

### Technical Problem

An aspect of the present invention is to provide a hypotonic composition for fat decomposition and a method for preparing the same, wherein a hypotonic composition for fat decomposition is prepared to contain sodium deoxycholate, hyaluronidase, and the like in a hypotonic solution composed of distilled water and sodium chloride.

Another aspect of the present invention is to provide a hypotonic composition for fat decomposition and a method for preparing the same, wherein a hypotonic composition for fat decomposition is prepared to contain sodium deoxycholate, hyaluronidase, a buffer, an additive, and the like in a hypotonic solution composed of distilled water and sodium chloride.

### Technical Solution

In accordance with an aspect of the present invention, there is provided a hypotonic composition for fat decomposition, the composition including: 50-3,000 I.U/ml of hyaluronidase; relative to the total weight of the hypotonic composition for fat decomposition, 0.01-9.9 wt% of sodium deoxycholate for selectively disrupting fat cells to reduce the volume of fat when the hypotonic composition for fat decomposition is injected into a subcutaneous layer; and a balance being a hypotonic solution for inducing physical decomposition of fat cells by an osmotic pressure difference.

In accordance with the present invention, the hypotonic composition for fat decomposition, may further comprise 0.01-50 wt% of a buffer for adjusting the pH of the hypotonic composition for fat composition, and 0.01-50 wt% of an additive for maintaining the period of pharmaceutical effect of the hypotonic composition for fat decomposition.

The hypotonic solution may be prepared by adding 0.1-8.9 g of sodium chloride into 1000 ml of distilled water, followed by stirring.

The buffer may include at least one of lactose hydrate, succinate, acetate, phosphate, citrate, aconitate, malate, and carbonate.

The additive may include at least one of a diluent, an adjuvant, an excipient, and a vehicle.

In accordance with another aspect of the present invention, there is provided a method for preparing a hypotonic composition for fat decomposition, the method including: preparing a hypotonic solution by adding sodium hydrochloride into distilled water, followed by stirring, the sodium hydrochloride inducing the physical decomposition of fat cells by an osmotic pressure difference when the hypotonic composition for fat decomposition is injected into a subcutaneous layer; preparing mixture solution A by adding sodium deoxycholate into the prepared hypotonic solution, followed by stirring, the sodium deoxycholate selectively disrupting fat cells to reduce the volume of fat when the hypotonic composition for fat decomposition is injected into a subcutaneous layer; and preparing the hypotonic composition for fat decomposition by adding hyaluronidase into the prepared mixture solution A, followed by stirring, the hyaluronidase increasing the permeability of a drug into subcutaneous connective tissue to allow the sodium deoxycholate to attain dispersion, delivery, and effect on fat cells.

The hypotonic composition for fat decomposition may include 50-3,000 I.U/ml of hyaluronidase, 0.01-9.9 wt% of sodium deoxycholate, and the balance being the hypotonic solution.

The method may further include adding into the hypotonic composition for fat decomposition a buffer for adjusting the pH of the hypotonic composition for fat decomposition and an additive for maintaining the period of pharmaceutical effect of the hypotonic composition for fat decomposition.

The hypotonic composition for fat decomposition may include 50-3,000 I.U/ml of the hyaluronidase; 0.01-9.9 wt% of the sodium deoxycholate; 0.01-50 wt% of the buffer; 0.01-50 wt% of the additive; and the balance being a hypotonic solution for inducing physical decomposition of fat cells by an osmotic pressure difference.

### Advantageous Effects

According to the present invention, a hypotonic composition for fat decomposition is prepared to contain sodium deoxycholate, hyaluronidase, and the like in a hypotonic solution composed of distilled water and sodium hydrochloride, so that the hypotonic composition for fat decomposition has effects of: reducing the occurrence of consolidation at an injection site due to the use of sodium deoxycholate alone; reducing the effect on fat cells and other cells containing fat components (including, e.g., nerve cells, muscle cells, bone cells, and the like) to reduce adverse effects, such as neural nerve palsy, muscle atrophy, and bone atrophy, compared with the use of sodium deoxycholate alone; reducing adverse effects, such as edema (swelling), erythema, and pain; increasing the efficiency of fat cell disruption through the combinative use of hyaluronidase based on a hypotonic solution; and allowing a use as a medicine for reducing fat accumulated in a topical site through subcutaneous administration.

Furthermore, according to the present invention, a hypotonic composition for fat decomposition is prepared to contain sodium deoxycholate, hyaluronidase, a buffer, an additive, and the like in a hypotonic solution composed of distilled water and sodium hydrochloride, so that sodium deoxycholate is effectively delivered into fat cells in the tissue injected with the hypotonic composition for fat decomposition, thereby attaining efficient fat decomposition using only a low dose of sodium deoxycholate and reducing edema, erythema, pain, and skin consolidation at an injected site due to the injection of a high dose of sodium deoxycholate.

### Brief Description of the Drawings

FIG. 1 is a flow chart showing a method for preparing a hypotonic composition for fat decomposition according to a first embodiment of the present invention.
FIG. 2 is a flow chart showing a method for preparing a hypotonic composition for fat decomposition according to a second embodiment of the present invention.
FIG. 3 is a graph showing relative weights of an example and comparative examples in a hypotonic composition for fat decomposition according to an embodiment of the present invention.

### Mode for Carrying Out the Invention

Technical terms used in this specification are used to describe only specific embodiments, and it is to be noted that the terms are not intended to limit the present invention. Furthermore, the technical terms used in the present invention should be interpreted as having meanings that are commonly understood by a person having ordinary skill in the art to which the present invention belongs, unless specifically defined in this specification, and should not be interpreted as having excessively comprehensive meanings or excessively reduced meanings. Furthermore, if the technical terms used in this specification are erroneous technical terms that do not precisely represent the spirit of the present invention, they should be replaced with technical terms that may be correctly understood by a person having ordinary skill in the art. Furthermore, common terms used in the present invention should be interpreted according to the definitions of dictionaries or according to the context and should not be interpreted as having excessively reduced meanings.

A singular number expression used in the present invention may include a plural number expression unless mentioned clearly and differently in context. In the present invention, terminology such as "composed" or "include" and the like should be construed not as necessarily including various elements or steps written in the present invention but as including the elements or steps in part or further including additional elements or steps.

Moreover, terminologies, which include an ordinal number, such as 1st or 2nd, may be used to describe various elements, but various elements should not be limited by the corresponding terminologies, respectively. The terminologies are only used for the purpose of discriminating one element from other elements. For example, a first element may be referred to as a second element and *vice versa* without departing from the scope of the present invention, and similarly, the 2nd element may be named the 1st element.

Hereinafter, preferred embodiments according to the present invention are described in detail with reference to the accompanying drawings. The same or similar elements are assigned the same reference numerals irrespective of reference numerals, and a redundant description thereof is omitted.

Furthermore, in describing the present invention, a detailed description of the known functions and constructions will be omitted if it is deemed to make the gist of the present invention unnecessarily vague. Furthermore, the accompanying drawings are provided to help easily understand the spirit of the present invention.

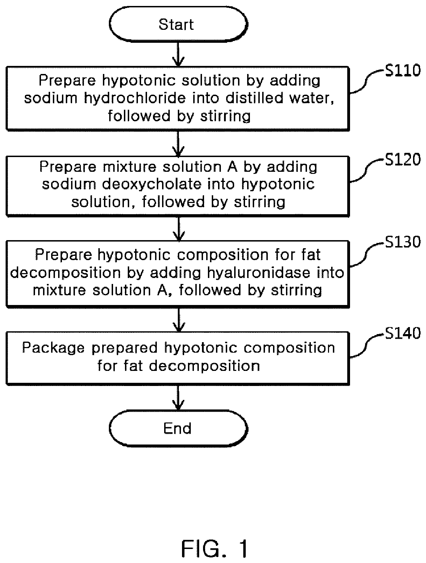

Hereinafter, a method for preparing a hypotonic composition for fat decomposition according to the present invention will be described in detail with reference to FIGS. 1 to 3.

FIG. 1 is a flow chart showing a method for preparing a hypotonic composition for fat decomposition according to a first embodiment of the present invention.

First, a hypotonic solution is prepared by adding sodium chloride into distilled water, followed by stirring.

The sodium hydrochloride is used for preparing a low-osmotic pressure solution (or hypotonic solution).

That is, the sodium chloride has an effect of inducing the physical disruption (decomposition) of fat cells by an osmotic pressure difference when the hypotonic composition for fat decomposition is subcutaneously injected.

Here, the distilled water may be contained in 1-99 wt% relative to the total weight of the hypotonic composition for fat decomposition, and the sodium chloride may be contained in 0.01-0.89 wt% (generally 0.45 wt%) relative to the total weight of the hypotonic composition for fat decomposition.

Here, the conditions and time for stirring may be such that stirring is carried out for 5-10 minutes (generally 10 min) in a stirring machine (not shown) with a rotation speed of 250-300 rpm while the pressure may be an atmospheric pressure and the temperature may be room temperature. The conditions and time for stirring may be variously set depending on the design of a designer. In addition, the mixture solutions may be prepared under the above stirring environment (the above conditions and time for stirring) in all the stirring steps below (S110).

Thereafter, mixture solution A is prepared by adding sodium deoxycholate into the prepared hypotonic solution, followed by stirring.

The sodium deoxycholate selectively disrupts fat cells to reduce the volume of fat when the hypotonic composition for fat decomposition is subcutaneously injected. The disrupted fat cells cannot accumulate fat cells again, leading to a permanent reduction in the volume of fat.

Here, the sodium deoxycholate may be contained in 0.01-9.9 wt% (generally 0.075 wt%) relative to the total weight of the hypotonic composition for fat decomposition (S120).

Thereafter, the hypotonic composition for fat decomposition is prepared by adding hyaluronidase into the prepared mixture solution A, followed by stirring.

Hyaluronidases are a huge family of enzymes that decomposes hyaluronic acid. The hyaluronic acid is a core component for an extracellular matrix and is a main member for an interstitial barrier. The hyaluronidase catalyzes the hydrolysis of the hyaluronic acid to lower the viscosity of the hyaluronic acid, thereby increasing tissue permeability and diffusion. Therefore, the hyaluronidase is used as a spreader or a dispersant together with other reagents, drugs, proteins, and the like, to improve dispersibility and delivery property.

That is, the hyaluronidase enhances the permeability of a drug through subcutaneous connective tissue by promoting (or catalyzing) the hydrolysis of the hyaluronic acid, thereby helping sodium deoxycholate, which is contained in the hypotonic composition for fat decomposition, to attain effective dispersion, delivery, and effect on fat cells.

In addition, the hyaluronidase includes hyaluronidase derived from bacteria (EC 4.2.99.1), hyaluronidase derived from leeches, other parasites, and crustaceans (EC 3.2.1.36), mammal type hyaluronidase (EC 3.2.1.35), and the like according to the action mechanism.

In addition, the hyaluronidase described herein is not limited thereto, and may include hyaluronidases originated from non-humans and hyaluronidases originated from humans.

That is, the hyaluronidase may include hyaluronidases originated from non-humans, such as rats, cats, rabbits, birds, cows, sheep, pigs, horses, fish, frogs, fungi, leeches, other parasites, crustaceans, and the like.

For example, the hyaluronidases originated from non-humans include hyaluronidases derived from cows, wasps, bees, white face hornets, Asian giant hornets, mice, pigs, rats (white rats), rabbits, sheep, orangutans, Philippine monkeys, guinea pigs, *Staphylococcus aureus,* streptococcus, and *Clostridium perfringens.*

In addition, the hyaluronidase originated from humans includes HYAL1, HYAL2, HYAL3, HYAL4, PH20, and the like.

In addition, the hyaluronidase includes sheep and cow PH20, soluble human PH20, soluble rHuPH20, and the like.

In addition, the hyaluronidase having a hyaluronidase biological activity of 50-3,000 I.U/ml (generally 90 I.U/ml) at room temperature may be added into the mixture solution A.

As such, the hypotonic composition for fat decomposition may be prepared by stirring the distilled water, the sodium chloride, the sodium deoxycholate, the hyaluronidase, and the like.

**[Table 1]**

| Ingredients | Composition ratio (wt%) |
|---|---|
| Distilled water | 1-99 |
| Sodium chloride | 0.01-0.89 |
| Sodium deoxycholate | 0.01-9.9 |
| Hyaluronidase | 50-3,000 I.U/ml |

In addition, the hypotonic composition for fat decomposition may be formed at a composition ratio (wt%) as shown in Table 1 above.

In addition, the hypotonic composition for fat decomposition is composed of: 50-3,000 I.U/ml of hyaluronidase; relative to the total weight of the hypotonic composition for fat decomposition, 0.01-9.9 wt% of sodium deoxycholate; and the balance being a hypotonic solution. Here, the hypotonic solution is for inducing physical disruption (or decomposition) of fat cells by an osmotic pressure difference when the hypotonic composition for fat decomposition is subcutaneously injected. The hypotonic solution is composed of 0.1-8.9 g (generally 4.5 g) of sodium hydrochloride relative to 1000 ml of the hypotonic solution and the balance being distilled water.

In addition, the hypotonic composition for fat decomposition is managed (or formed/prepared) as a solution dosage form or freeze-dried dosage form.

In addition, a freeze-dried dosage form of the hypotonic composition for fat decomposition may be prepared in a liquid state by a diluent (S130).

Thereafter, the prepared hypotonic composition for fat decomposition is packaged in a predetermined container (not shown) for each use (each unit). Here, the packaged hypotonic composition for fat decomposition containing a pharmaceutical active compound needs to be sterile.

That is, the prepared hypotonic composition for fat decomposition is packaged in an ample, vial, a needle syringe, or the like.

In addition, the packaged hypotonic composition for fat decomposition is directly injected into a subcutaneous layer of the user body to selectively disrupt the fat cells, thereby inducing a reduction in the volume of fat and reducing and correcting the face and body. Here, the hypotonic composition for fat decomposition may be injected into a subcutaneous fat layer of a double chin area of a user.

That is, when the hypotonic composition for fat decomposition is injected into the body tissue, cells (including, for example, fat cells) of the tissue injected with the hypotonic composition for fat decomposition become swollen by an osmotic pressure difference, and the cells swollen by the osmotic pressure difference allow a more effective disruption of fat cells by sodium deoxycholate.

As described above, the hypotonic composition for fat decomposition is directly injected into a double chin area with relatively few motor nerves of a user, thereby maximizing the fat decomposition effect and reducing adverse effects due to the permanent damage to motor nerves.

Through an action of the hypotonic composition for fat decomposition injected into the subcutaneous fat layer, fat cell membranes are disrupted and fat matter is changed to be water-soluble. Thereafter, the water-soluble fat matter may be discharged from the body by urine or sweat (S140).

As described above, in an example of the present invention, the hypotonic composition for fat decomposition did not contain phosphatidylcholine, which is involved in the occurrence of adverse effects.

Whereas, the hypotonic composition for fat decomposition contained sodium deoxycholate proved to have a fat decomposition effect.

In addition, the hypotonic composition for fat decomposition contains the hyaluronidase based on the hypotonic solution, thereby maximizing the fat decomposition effect of the sodium deoxycholate and reducing inconvenience, such as edema, erythema, pain, and skin consolidation.

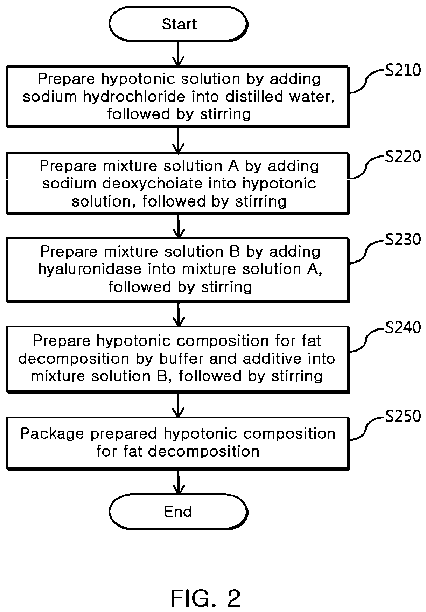

FIG. 2 is a flow chart showing a method for preparing a hypotonic composition for fat decomposition according to a second embodiment of the present invention.

First, a hypotonic solution is prepared by adding sodium chloride into distilled water, followed by stirring.

The sodium hydrochloride is used for preparing a low-osmotic pressure solution (or hypotonic solution).

That is, the sodium chloride has an effect of inducing the physical disruption (decomposition) of fat cells by an osmotic pressure difference when the hypotonic composition for fat decomposition is subcutaneously injected.

Here, the distilled water may be contained in 1-99 wt% relative to the total weight of the hypotonic composition for fat decomposition, and the sodium chloride may be contained in 0.01-0.89 wt% (generally 0.45 wt%) relative to the total weight of the hypotonic composition for fat decomposition.

Here, the stirring conditions and time may be such that stirring is carried out for 5-10 minutes (generally 10 min) in a stirring machine (not shown) with a rotation speed of 250-300 rpm while the pressure may be an atmospheric pressure and the temperature may be room temperature. The conditions and time for stirring may be variously set depending on the design of a designer. In addition, the mixture solutions may be prepared under the above stirring environment (the above conditions and time for stirring) in all the stirring steps below (S210).

Thereafter, mixture solution A is prepared by adding sodium deoxycholate into the prepared hypotonic solution, followed by stirring.

The sodium deoxycholate selectively disrupts fat cells to reduce the volume of fat when the hypotonic composition for fat decomposition is subcutaneously injected. The disrupted fat cells cannot accumulate fat cells again, leading to a permanent reduction in the volume of fat.

Here, the sodium deoxycholate may be contained in 0.01-9.9 wt% (generally 0.075 wt%) relative to the total weight of the hypotonic composition for fat decomposition (S220).

Thereafter, mixture solution B is prepared by adding hyaluronidase into the prepared mixture solution A, followed by stirring.

In addition, the hyaluronidase having a hyaluronidase biological activity of 50-3,000 I.U/ml (generally 90 I.U/ml) at room temperature may be added into the mixture solution A (S230).

Thereafter, the hypotonic composition for fat decomposition is prepared by adding a buffer and other additives into the prepared mixture B, followed by stirring.

The buffer is used for adjusting the pH of the hypotonic composition for fat decomposition, and includes lactose hydrate, succinate, acetate, phosphate, citrate, aconitate, malate, carbonate, and the like.

Besides the buffer described in an embodiment of the present invention, various buffers that provide acceptable pH stability or satisfy buffer capacity within an indicated (or predetermined) range may be used.

The additive is used for maintaining the period of pharmaceutical effect of the hypotonic composition for fat decomposition, and includes a carrier, such as an adjuvant, an excipient, or a vehicle (medium/intermediate).

Here, a pharmaceutical carrier may be a sterile liquid, water, and oil including a plant- or synthesis-originated carrier, such as petroleum, animal, peanut oil, soybean oil, mineral oil, and sesame oil.

In addition, a saline solution, aqueous dextrose, a glycerol solution, and the like may be used as a liquid carrier.

For example, a pharmaceutically acceptable carrier used for use in a parenteral preparation includes an aqueous vehicle, a non-aqueous vehicle, an antimicrobial agent, a tonicity agent, an antioxidant, a topical anesthetic, a suspending and dispersing agent, an emulsifying agent, a chelating agent, and other pharmaceutically acceptable materials.

The aqueous vehicle includes a sodium chloride injection, a Ringer injection, an isotonic dextrose injection, a sterile injection, a dextrose and lactate Ringer injection, and the like.

The non-aqueous vehicle (or non-aqueous parenteral vehicle) includes a plant-originated fixed oil, cottonseed oil, corn oil, sesame oil, peanut oil, and the like.

The antimicrobial agent in a bacteriostatic or bactericidal bacterial concentrate includes phenol or cresol, benzyl alcohol, chlorobutanol, methyl and propyl p-hydroxybenzoic ester, thimerosal, benzalkonium chloride, benzethonium chloride, and the like.

The tonicity agent includes sodium chloride, dextrose, and the like.

The antioxidant includes sodium bisulfate, and the like.

The topical anesthetic includes lidocaine hydrochloride, procaine hydrochloride, and the like.

The suspending and dispersing agent includes sodium carboxymethylcellulose, hydroxypropyl methylcellulose, polyvinylpyrrolidone, and the like.

The emulsifying agent includes polysorbate 80 (TWEENs 80), and the like.

The metal ion chelating agent includes ethylenediaminetetraacetic acid (EDTA) and the like.

The pharmaceutical carrier may include: ethyl alcohol, polyethylene glycol, and propylene glycol for a water-miscible vehicle; sodium hydroxide, hydrochloric acid, citric acid, and lactic acid for pH adjustment; and the like.

In addition, the additive may include a diluent (including, for example, lactose, sucrose, dicalcium phosphate, carboxymethylcellulose, and the like), a lubricant (including, e.g., magnesium stearate, calcium stearate, talc, and the like), a binder (including, e.g., starch, natural gum, and the like), and the like.

In addition, the additive may include a small amount of wetting agent or emulsifying agent, a pH buffer (including, e.g., acetate, sodium citrate, a cyclodextrin derivative, sorbitan monooleate, triethanolamine sodium acetate, triethanolaminolate, etc.) and the like.

Here, the content of the buffer may be 0.01-50 wt% relative to the total weight of the hypotonic composition for fat decomposition, and the content of the additive (or other additive) may be 0.01-50 wt% relative to the total weight of the hypotonic composition for fat decomposition.

As such, the hypotonic composition for fat decomposition may be prepared by stirring the distilled water, the sodium chloride, the sodium deoxycholate, the hyaluronidase, the buffer, the additive, and the like.

**[Table 2]**

| Ingredients | Composition ratio (wt%) |
|---|---|
| Distilled water | 1-99 |
| Sodium chloride | 0.01-0.89 |
| Sodium deoxycholate | 0.01-9.9 |
| Hyaluronidase | 50-3,000 I.U/ml |
| Buffer | 0.01-50 |
| Additive | 0.01-50 |

In addition, the hypotonic composition for fat decomposition may be formed at a composition ratio (wt%) as shown in Table 2 above.

In addition, the hypotonic composition for fat decomposition contains: 50-3,000 I.U/ml of hyaluronidase; relative to the total weight of the hypotonic composition for fat decomposition, 0.01-9.9 wt% of sodium deoxycholate for selectively disrupting fat cells to reduce the volume of fat when the hypotonic composition for fat decomposition is injected into a subcutaneous layer, 0.01-50 wt% of a buffer for adjusting the pH of the hypotonic composition for fat composition, and 0.01-50 wt% of an additive for maintaining the period of pharmaceutical effect of the hypotonic composition for fat decomposition; and the balance being a hypotonic solution for inducing physical decomposition of fat cells by an osmotic pressure difference. Here, the hypotonic solution is for inducing physical disruption (or decomposition) of fat cells by an osmotic pressure difference when the hypotonic composition for fat decomposition is subcutaneously injected. The hypotonic solution is composed of, relative to 1000 ml of the hypotonic solution, 0.1-8.9 g (generally 4.5 g) of sodium hydrochloride, and the balance being distilled water.

In addition, the hypotonic composition for fat decomposition is managed (or formed/prepared) as a solution dosage form or freeze-dried dosage form.

In addition, a freeze-dried dosage form of the hypotonic composition for fat decomposition may be prepared in a liquid state by a diluent (S240).

Thereafter, the prepared hypotonic composition for fat decomposition is packaged in a predetermined container (not shown) for each use (or each unit). Here, the packaged hypotonic composition for fat decomposition containing a pharmaceutical active compound needs to be sterile.

That is, the prepared hypotonic composition for fat decomposition is packaged in an ample, vial, a needle syringe, or the like.

In addition, the packaged hypotonic composition for fat decomposition is directly injected into a subcutaneous layer of the user body to selectively disrupt the fat cells, thereby inducing a reduction in the volume of fat and reducing and correcting the face and body. Here, the hypotonic composition for fat decomposition may be injected into the subcutaneous fat layer of a double chin area of a user.

That is, when the hypotonic composition for fat decomposition is injected into the body tissue, cells (including, for example, fat cells) of the tissue injected with the hypotonic composition for fat decomposition become swollen by an osmotic pressure difference, and the cells swollen by an osmotic pressure difference allow a more effective disruption of fat cells by sodium deoxycholate.

As described above, the hypotonic composition for fat decomposition is directly injected into a double chin area with relatively few motor nerves of a user, thereby maximizing the fat decomposition effect and reducing adverse effects due to the permanent damage to motor nerves.

Through an action of the hypotonic composition for fat decomposition injected into the subcutaneous fat layer, fat cell membranes are disrupted and fat matter is changed to be water-soluble. Thereafter, the water-soluble fat matter may be discharged from the body by urine or sweat (S250).

### <Example>

The hypotonic composition (injection composition) for fat decomposition according to an embodiment of the present invention employed, relative to a total amount of 100 ml, 9000 I.U of hyaluronidase, 75 mg of sodium deoxycholate, 450 mg of sodium chloride, and the balance being distilled water.

### <Comparative Example 1>

Physiological saline was used alone.

### <Comparative Example 2>

Physiological saline and a high dose of sodium deoxycholate (1000 mg) were used.

### <Comparative Example 3>

Physiological saline and 75 mg of sodium deoxycholate were used.

### <Comparative Example 4>

Physiological saline and 9000 I.U of hyaluronidase were used.

### <Comparative Example 5>

A hypotonic solution was used alone.

### <Comparative Example 6>

Physiological saline, 9000 I.U of hyaluronidase, and 75 mg of sodium deoxycholate were used.

The compositions of the injection composition according to an example of the present invention and the injection compositions according to Comparative Examples 1 to 6 may be shown in Table 3 below.

**[Table 3]**

| Ingredients | Example | Comparative Example 1 | Comparative Example 2 | Comparative Example 3 | Comparative Example 4 | Comparative Example 5 | Comparative Example 6 |
|---|---|---|---|---|---|---|---|
| Hyaluronidase | 9000I.U | - | - | - | 9000I.U | - | 9000I.U |
| Sodium deoxycholate | 75mg (0.075%) | - | 1000mg (1%) | 75mg (0.075%) | - | - | 75mg (0.075%) |
| Sodium chloride | 450mg (0.45%) | 900mg (0.9%) | 900mg (0.9%) | 900mg (0.9%) | 900mg (0.9%) | 450mg (0.45%) | 900mg (0.9%) |
| Distilled water | Balance | Balance | Balance | Balance | Balance | Balance | Balance |
| Total | 100ml | 100ml | 100ml | 100ml | 100ml | 100ml | 100ml |

Here, all the injection compositions were prepared to have a volume of 100 ml. Here, "Hyallap" (1500 I.U per vial) by Wooridul Pharmaceutical Ltd. was used as the hyaluronidase. For the compositions of the injection compositions, sodium deoxycholate (Sigma-Aldrich) was weighed according to the respective contents using a microbalance, and dissolved in physiological saline (Dai Han Pharm.) or a hypotonic solution containing mixed physiological saline and distilled water (Dai Han Pharm.), thereby preparing the respective injection compositions according to corresponding contents.

In addition, each of the injection compositions containing hyaluronidase as shown in Example, Comparative Example 4, and Comparative Example 6, was prepared by adding each of the prepared solutions into a "Hyallap" vial to dissolve freeze-dried hyaluronidase, and followed by collection.

### <Test Example>

In the present test, 5-week-old female Male Sprague Dawley rats as experimental animals were purchased from Orient Biotech. In addition, rat breeding environmental conditions were set to a temperature of 22-24°C and relative humidity of 30-50%, and two rats per breeding box were bred. In addition, 12-hour light-dark cycle was conditioned with an illumination of 150-300 Lux given from 7:00 AM to 7:00 PM. A high-fat diet (containing 45% fat, Research Diet Inc., USA) as a feed was freely accessible. The animals having obesity induced with a high-fat diet for 8 weeks were grouped into two groups based on the body weight, and after the grouping, the subcutaneous inguinal fat of the right side of each animal was subcutaneously injected with the injection of the present invention (or Example) and the injections of Comparative Examples 1-6 for 4 weeks.

**[Table 4]**

| Injection composition | Group | Feed | Dose (ml) | Number of individuals |
|---|---|---|---|---|
| Comparative Example 1 | Normal control group | General feed | 500 | 8 |
| Comparative Example 1 | Obesity-induced control group | High-fat feed | 500 | 8 |
| Comparative Example 2 | Test group 1 | High-fat feed | 500 | 8 |
| Comparative Example 3 | Test group 2 | High-fat feed | 500 | 8 |
| Comparative Example 4 | Test group 3 | High-fat feed | 500 | 8 |
| Comparative Example 5 | Test group 4 | High-fat feed | 500 | 8 |
| Comparative Example 6 | Test group 5 | High-fat feed | 500 | 8 |
| Example | Test group 6 | High-fat feed | 500 | 8 |

The injection was administered once a week for four weeks. Two weeks after the final injection, each animal was sacrificed by inhalation anesthesia with isoflurane. Then, the inguinal fat at left and right sides was extracted, and the extracted fat was weighed to calculate the relative weight of fat tissue by dividing the fat weight by the body weight of the corresponding animal.

All test results obtained in the test were expressed as mean ± standard error and analyzed using SPSS (version 20, IBM SPSS Statistics, USA). In addition, Levene's test for comparing homogeneity of variance was performed on all data, and when the variance was homogeneous, one-one-way Analysis of Variance (ANOVA) was performed. When significance was observed, Dunnett's T-test was performed to find out a test group having a significant difference with a control group. The number of individuals per group was 8, and statistical significance was determined and marked with an asterisk (* or **). (Significance level *: 5% for both sides, **: 1%).

As a result of measuring the extracted inguinal fat, the relative inguinal fat weights in the obesity-induced control group (or obesity control group) were significantly higher than those in the normal control group, and thus, it could be observed that the inguinal fat caused by obesity induction was largely formed.

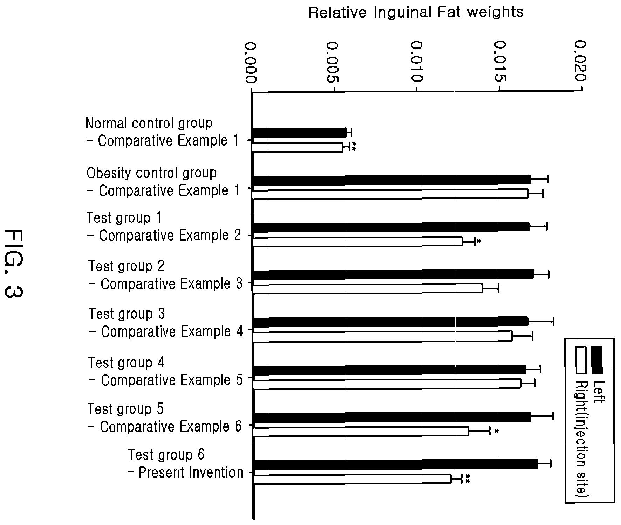

As shown in FIG. 3, as a result of subcutaneous injection into obese animals with the injection according to Example of the present invention and the injections of Comparative Examples 1-6 once per week for 4 weeks, a remarkable difference between the left and right inguinal fat was observed in the injections of Comparative Examples 2, 3, and 6 and the injection of Example of the present invention.

In addition, as shown in FIG. 3, the injections according to Example of the present invention and Comparative Examples 2 and 6 showed a significantly low right relative inguinal fat weight when compared with the obesity-induced control.

Especially, as shown in Table 5 below, the right relative inguinal fat weight in the group administered with the injection according to Example of the present invention was 0.0120 ± 0.0019, which was about 28.1% reduced compared with the right relative inguinal fat weight in the obesity-induced control group, 0.0167 ± 0.0026, showing the lowest relative inguinal fat weight.

**[Table 5]**

| Injection composition | Group | Left relative inguinal fat weight (Left inguinal fat weight/body weight) | Right relative inguinal fat weight (Right inguinal fat weight/body weight) | Reduction in fat compared with obesity-induced control group |
|---|---|---|---|---|
| Comparative Example 1 | Normal control group | 0.0057 ± 0.0008 | 0.0055 ± 0.0010** | - |
| Comparative Example 1 | Obesity-induced group | 0.0169 ±0.0030 | 0.0167 ± 0.0026 | - |
| Comparative Example 2 | Test group 1 | 0.0168 ±0.0031 | 0.0128 ± 0.0020 | 23.3% |
| Comparative Example 3 | Test group 2 | 0.0170 ±0.0026 | 0.0139 ± 0.0028 | 16.7% |
| Comparative Example 4 | Test group 3 | 0.0167 ±0.0044 | 0.0157 ± 0.0035 | 5.9% |
| Comparative Example 5 | Test group 4 | 0.0165 ±0.0025 | 0.0162 ±0.0025 | 2.9% |
| Comparative Example 6 | Test group 5 | 0.0168 ±0.0038 | 0.0130 ±0.0038* | 22.1% |
| Example | Test group 6 | 0.0172 ±0.0023 | 0.0120 ±0.0019** | 28.1% |

As described above, when compared with the use of a high dose of sodium deoxycholate or hyaluronidase alone in a physiological saline solution or the use of sodium deoxycholate and hyaluronidase in a physiological saline solution, the use of sodium deoxycholate and hyaluronidase in a hypotonic solution according to the example of the present invention was confirmed to have an excellent topical fat decomposition effect.

The embodiments of the present invention have been described that in order to maximize a fat decomposition effect of the hypotonic composition for fat decomposition and reduce sides effects due to the permanent damage to motor nerve, the hypotonic composition for fat decomposition is directly injected into the double-chin area of a user, but is not limited thereto, and thus, from the stability test depending on the composition ratio of sodium deoxycholate and hyaluronidase contained in the hypotonic composition for fat decomposition on a face part (including, e.g., a zygomatic site, cheek, orbital fat, etc.), and a body part (including, e.g., abdomen, back, side, upper arm, thigh, calf, etc.), the hypotonic composition for fat decomposition can also be injected into the face part, the body part, and the like.

As set forth above, in the embodiments of the present invention, a hypotonic composition for fat decomposition is prepared to contain sodium deoxycholate, hyaluronidase, and the like in a hypotonic solution composed of distilled water and sodium hydrochloride, so that the hypotonic composition has effects of reducing the occurrence of consolidation at an injection site due to the use of a high dose (e.g., 10-95 wt% relative to the total weight of the composition) of sodium deoxycholate alone, reducing the effect on fat cells and other cells containing fat components (including, e.g., nerve cells, muscle cells, bone cells, and the like) to reduce adverse effects, such as neural nerve palsy, muscle atrophy, and bone atrophy, compared with the use of sodium deoxycholate alone, reducing adverse effects, such as edema (swelling), erythema, and pain, increasing the efficiency of fat cell disruption through the combinative use of hyaluronidase based on a hypotonic solution, and allowing the use as a medicine for reducing fat accumulated in a topical site through subcutaneous administration.

Furthermore, as set forth above, in the embodiments of the present invention, a hypotonic composition for fat decomposition is prepared to contain sodium deoxycholate, hyaluronidase, a buffer, an additive, and the like in a hypotonic solution composed of distilled water and sodium hydrochloride, so that sodium deoxycholate is effectively delivered into fat cells in the tissue injected with the hypotonic composition for fat decomposition, thereby achieving an efficient fat decomposition effect using only a low-dose (e.g., 0.01-9.9 w% relative to the total weight of the hypotonic composition for fat decomposition) of sodium deoxycholate, and reducing edema, erythema, pain, and skin consolidation at an injected site due to the injection of a high concentration of sodium deoxycholate.

### Industrial applicability

According to the present invention, a hypotonic composition for fat decomposition is prepared to contain sodium deoxycholate, hyaluronidase, and the like in a hypotonic solution composed of distilled water and sodium hydrochloride, so that the hypotonic composition has effects of reducing the occurrence of consolidation at an injection site due to the use of sodium deoxycholate alone, reducing the effect on fat cells and other cells containing fat components (including, e.g., nerve cells, muscle cells, bone cells, and the like) to reduce adverse effects, such as neural nerve palsy, muscle atrophy, and bone atrophy, compared with the use of sodium deoxycholate alone, reducing adverse effects, such as edema (swelling), erythema, and pain, increasing the efficiency of fat cell disruption through the combinative use of hyaluronidase based on a hypotonic solution, and allowing the use as a medicine for reducing fat accumulated in a topical site through subcutaneous administration, so that the present invention can be widely used in the fields of skin care, medicine, and the like.

## Claims

1. A hypotonic composition for fat decomposition, the composition comprising: 50-3,000 I.U/ml of hyaluronidase; relative to the total weight of the hypotonic composition for fat decomposition, 0.01-9.9 wt% of sodium deoxycholate for selectively disrupting fat cells to reduce the volume of fat when the hypotonic composition for fat decomposition is injected into a subcutaneous layer; and a balance being a hypotonic solution for inducing physical decomposition of fat cells by an osmotic pressure difference.

2. The composition of claim 1, further comprising 0.01-50 wt% of a buffer for adjusting the pH of the hypotonic composition for fat decomposition, and 0.01-50 wt% of an additive for maintaining the period of pharmaceutical effect of the hypotonic composition for fat decomposition.

3. The composition of claim 1 or 2, wherein the hypotonic solution is prepared by adding 0.1-8.9 g of sodium chloride into 1000 ml of distilled water, followed by stirring.

4. The composition of claim 2, wherein the buffer comprises at least one of lactose hydrate, succinate, acetate, phosphate, citrate, aconitate, malate, and carbonate.

5. The composition of claim 2, wherein the additive comprises at least one of a diluent, an adjuvant, an excipient, and a vehicle.

6. A method for preparing a hypotonic composition for fat decomposition, the method comprising:
preparing a hypotonic solution by adding sodium hydrochloride into distilled water, followed by stirring, the sodium hydrochloride inducing the physical decomposition of fat cells by an osmotic pressure difference when the hypotonic composition for fat decomposition is injected into a subcutaneous layer;
preparing mixture solution A by adding sodium deoxycholate into the prepared hypotonic solution, followed by stirring, the sodium deoxycholate selectively disrupting fat cells to reduce the volume of fat when the hypotonic composition for fat decomposition is injected into a subcutaneous layer; and
preparing the hypotonic composition for fat decomposition by adding hyaluronidase into the prepared mixture solution A, followed by stirring, the hyaluronidase increasing the permeability of a drug into subcutaneous connective tissue to allow the sodium deoxycholate to attain dispersion, delivery, and effect on fat cells.

7. The method of claim 6, wherein the hypotonic composition for fat decomposition comprises 50-3,000 I.U/ml of hyaluronidase, 0.01-9.9 wt% of sodium deoxycholate, and the balance being the hypotonic solution.

8. The method of claim 6, the method further comprising:
adding into the hypotonic composition for fat decomposition a buffer for adjusting the pH of the hypotonic composition for fat decomposition and an additive for maintaining the period of pharmaceutical effect of the hypotonic composition for fat decomposition.

9. The method of claim 8, wherein the hypotonic composition for fat decomposition comprises 50-3,000 I.U/ml of the hyaluronidase; 0.01-9.9 wt% of the sodium deoxycholate; 0.01-50 wt% of the buffer; 0.01-50 wt% of the additive; and the balance being a hypotonic solution for inducing physical decomposition of fat cells by an osmotic pressure difference.

## Patentansprüche

1. Hypotonische Zusammensetzung zur Fettzersetzung, die Zusammensetzung Folgendes umfassend: 50 bis 3.000 I.E./ml Hyaluronidase; bezogen auf das Gesamtgewicht der hypotonischen Zusammensetzung zur Fettzersetzung 0,01 bis 9,9 Gew.-% Natriumdeoxycholat zum selektiven Aufschließen von Fettzellen, so dass das Fettvolumen reduziert wird, wenn die hypotonische Zusammensetzung zur Fettzersetzung in eine subkutane Schicht injiziert wird; und wobei der Rest eine hypotonische Lösung zum Induzieren der physikalischen Zersetzung von Fettzellen durch eine osmotische Druckdifferenz ist.

2. Zusammensetzung nach Anspruch 1, ferner umfassend 0,01 bis 50 Gew.-% eines Puffers zum Einstellen des pH-Werts der hypotonischen Zusammensetzung zur Fettzersetzung und 0,01 bis 50 Gew.-% eines Additivs zur Aufrechterhaltung der Dauer der pharmazeutischen Wirkung der hypotonischen Zusammensetzung zur Fettzersetzung.

3. Zusammensetzung nach Anspruch 1 oder 2, wobei die hypotonische Lösung durch Zugeben von 0,1 bis 8,9 g Natriumchlorid in 1000 ml destilliertes Wasser und anschließendes Rühren hergestellt wird.

4. Zusammensetzung nach Anspruch 2, wobei der Puffer mindestens eines aus Laktosehydrat, Succinat, Acetat, Phosphat, Citrat, Aconitat, Malat und Carbonat umfasst.

5. Zusammensetzung nach Anspruch 2, wobei das Additiv mindestens eines aus einem Verdünnungsmittel, einem Adjuvans, einem Hilfsstoff und einem Vehikel umfasst.

6. Verfahren zum Herstellen einer hypotonischen Zusammensetzung zur Fettzersetzung, das Verfahren Folgendes umfassend:
Herstellen einer hypotonischen Lösung durch Zugeben von Natriumhydrochlorid in destilliertes Wasser und anschließendes Rühren, wobei das Natriumhydrochlorid die physikalische Zersetzung von Fettzellen durch eine osmotische Druckdifferenz induziert, wenn die hypotonische Zusammensetzung zur Fettzersetzung in eine subkutane Schicht injiziert wird;
Herstellen einer Mischungslösung A durch Zugeben von Natriumdeoxycholat in die hergestellte hypotonische Lösung und anschließendes Rühren, wobei das Natriumdeoxycholat selektiv Fettzellen aufschließt, so dass das Fettvolumen reduziert wird, wenn die hypotonische Zusammensetzung zur Fettzersetzung in eine subkutane Schicht injiziert wird; und
Herstellen der hypotonischen Zusammensetzung zur Fettzersetzung durch Zugeben von Hyaluronidase in die herstellte Mischungslösung A und anschließendes Rühren, wobei die Hyaluronidase die Permeabilität eines Arzneimittels in das subkutane Bindegewebe erhöht, so dass das Natriumdeoxycholat eine Dispersion, Abgabe und Wirkung auf Fettzellen erreichen kann.

7. Verfahren nach Anspruch 6, wobei die hypotonische Zusammensetzung zur Fettzersetzung 50 bis 3.000 I.E./ml Hyaluronidase, 0,01 bis 9,9 Gew.-% Natriumdeoxycholat umfasst und der Rest die hypotonische Lösung ist.

8. Verfahren nach Anspruch 6, das Verfahren ferner Folgendes umfassend:
zu der hypotonischen Zusammensetzung zur Fettzersetzung Zugeben eines Puffers zur Einstellung des pH-Wertes der hypotonischen Zusammensetzung zur Fettzersetzung und eines Additivs zur Aufrechterhaltung der Dauer der pharmazeutischen Wirkung der hypotonischen Zusammensetzung zur Fettzersetzung.

9. Verfahren nach Anspruch 8, wobei die hypotonische Zusammensetzung zur Fettzersetzung 50 bis 3.000 I.E./ml der Hyaluronidase; 0,01 bis 9,9 Gew.-% des Natriumdeoxycholats; 0,01 bis 50 Gew.-% des Puffers; 0,01 bis 50 Gew.-% des Additivs umfasst und der Rest eine hypotonische Lösung zum Induzieren der physikalischen Zersetzung von Fettzellen durch eine osmotische Druckdifferenz ist.

## Revendications

1. Une composition hypotonique de décomposition de graisse, la composition comprenant : 50 à 3000 IU/mL d'hyaluronidase par rapport au poids total de la composition hypotonique pour la décomposition de graisse, 0,01 à 9,9 % en poids de désoxycholate de sodium pour l'interruption sélective de cellules graisseuses afin de réduire le volume de graisse lorsque la composition hypotonique de décomposition de graisse est injectée dans une couche sous-cutanée, et un reliquat étant une solution hypotonique afin d'induire une décomposition physique de cellules graisseuses par une différence de pression osmotique.

2. La composition selon la revendication 1, comprenant en outre 0,01 à 50 % en poids d'un tampon permettant d'ajuster le pH de la composition hypotonique de décomposition de graisse, et 0,01 à 50 % en poids d'un additif permettant de maintenir la durée d'effet pharmaceutique de la composition hypotonique de décomposition de graisse.

3. La composition selon la revendication 1 ou 2, dans laquelle la solution hypotonique est préparée en ajoutant 0,1 à 8,9 g de chlorure de sodium dans 1000 mL d'eau distillée, puis en agitant ce mélange.

4. La composition selon la revendication 2, dans laquelle le tampon comprend au moins un parmi lactose hydraté, succinate, acétate, phosphate, citrate, aconitate, malate, et carbonate.

5. La composition selon la revendication 2, dans laquelle l'additif comprend au moins un parmi un diluant, un adjuvant, un excipient et un véhicule.

6. Un procédé de préparation d'une composition hypotonique de décomposition de graisse, le procédé comprenant :
la préparation d'une solution hypotonique en ajoutant de l'hydrochlorure de sodium dans de l'eau distillée, puis en agitant ce mélange, l'hydrochlorure de sodium induisant la décomposition physique de cellules graisseuses par une différence de pression osmotique lorsque la composition hypotonique de décomposition de graisse est injectée dans une couche sous-cutanée ;
la préparation de la solution de mélange A en ajoutant du désoxycholate de sodium dans la préparation de solution hypotonique, puis en agitant le mélange, le désoxycholate de sodium interrompant sélectivement les cellules graisseuses afin de réduire le volume de graisse lorsque la composition hypotonique de décomposition de graisse est injectée dans une couche sous-cutanée ; et
la préparation de la composition hypotonique de décomposition de graisse en ajoutant de l'hyaluronidase dans la préparation de solution de mélange A, puis en ajoutant le mélange, l'hyaluronidase augmentant la pénétration d'un médicament dans un issu conjonctif sous-cutané afin de permettre au désoxycholate de sodium de parvenir à réaliser la dispersion, l'administration et l'effet sur les cellules graisseuses.

7. Le procédé selon la revendication 6, dans lequel la composition hypotonique de décomposition de graisse comprend 50 à 3000 IU/mL d'hyaluronidase, 0,01 à 9,9 % en poids de désoxycholate de sodium et le reliquat étant la solution hypotonique.

8. Le procédé selon la revendication 6, comprenant en outre :
l'ajout dans la composition hypotonique de décomposition de graisse d'un tampon permettant d'ajuster le pH de la composition hypotonique de décomposition de graisse et d'un additif permettant de maintenir la durée d'effet pharmaceutique de la composition hypotonique de décomposition de graisse.

9. Le procédé selon la revendication 8, dans lequel la composition hypotonique de décomposition de graisse comprend 50 à 3000 IU/mL de l'hyaluronidase, 0,01 à 9,9 % en poids du désoxycholate de sodium, 0,01 à 50 % en poids du tampon, 0,01 à 50 % en poids de l'additif, et le reliquat étant une solution hypotonique afin d'induire une décomposition physique de cellules graisseuses par une différence de pression osmotique.
